# EUROPÄISCHE PATENTSCHRIFT

(11) **EP 2 425 896 B1**
(45) Veröffentlichungstag und Bekanntmachung des Hinweises auf die Patenterteilung: **29.07.2015**
(21) Anmeldenummer: 11006564.6
(22) Anmeldetag: 10.08.2011
(51) Int. Cl.: B01L 3/02, A61M 5/315

(54) **Spritze für den Gebrauch mit einer Dosiervorrichtung**
Syringe for use with a metering device
Seringue pour l'utilisation à l'aide d'un dispositif de dosage

(30) Priorität: 30.08.2010 DE 102010035891
(43) Veröffentlichungstag der Anmeldung: 07.03.2012
(73) Patentinhaber: Eppendorf AG, 22339 Hamburg (DE)
(72) Erfinder: Löhn, Jürgen, 27419 Gross Meckelsen (DE)
(74) Vertreter: Hauck Patentanwaltspartnerschaft mbB

(56) Entgegenhaltungen:
- US-A- 4 406 170
- US-A- 5 620 660
- US-A1- 2003 212 372

## Beschreibung

Die Erfindung bezieht sich auf eine Spritze mit Spritzenzylinder und Spritzenkolben für den Gebrauch mit einer Dosiervorrichtung, die eine Aufnahme für den Spritzenzylinder und eine axial verlagerbare Kolbenaufnahme für den Spritzenkolben aufweist.

Pipetten sind Dosiervorrichtungen zum Abmessen und Übertragen von Flüssigkeiten. Sie sind häufig als Repetier- oder Multipetten ausgeführt, welche gemeinsam mit einer Spritze verwendet werden, um Flüssigkeit in die Spritze aufzunehmen und schrittweise aus dieser abzugeben. Eine Repetierpipette ist aus der DE 29 26 691 C2 und der US 4 406 170 bekannt. Bei dieser ist die Spritzenaufnahme eine U-förmige Nut in einem Pipettengehäuse, in die ein Spritzenflansch durch eine seitliche Gehäuseöffnung eingeschoben werden kann. In der Aufnahme wird der Spritzenflansch von einer Druckfeder in Richtung der Abgabeöffnung belastet. Der Spritzenkolben hat einen zylindrischen Betätigungsabschnitt, der mittels einer Klemmvorrichtung in einem Aufnahmekörper fixierbar ist.

Eine Kolbenrückschiebeeinrichtung ist als Hebel des Aufnahmekörpers ausgeführt, der durch einen seitlichen Gehäuseschlitz herausragt. Durch Bewegen des Hebels vom Spritzenflansch weg ist der Kolben aus der Spritze herausziehbar. Die Kolbenvorschiebeeinrichtung ist eine Zahnstangen-Klinkeneinrichtung, wobei die Zahnstange mit dem Aufnahmekörper verbunden und die Klinke schwenkbar an einem hin- und herbewegbaren Antriebshebel gelagert ist. Beim Schwenken des Antriebshebels zum Spritzenflansch hin rastet die Klinke ein und nimmt die Zahnstange und den damit verbundenen Kolben in der gleichen Richtung mit. Beim Schwenken in Gegenrichtung springt die Klinke aus der sägezahnförmigen Zahnung heraus und die Kolbenstellung ändert sich nicht. Eine Schrittweiteneinstelleinrichtung weist eine mit einem Drehknopf gekoppelte Zunge auf, welche die Zahnung je nach Stellung des Drehknopfes mehr oder weniger überdeckt. Durch Einstellen des Drehknopfes ist der Abschnitt der Klinkenbewegung einstellbar, in dem diese in die Zahnstange eingreift und den Kolben mitnimmt. Damit ist das von der Spritze bei jedem Dosierschritt abgegebene Flüssigkeitsvolumen mittels des Drehknopfes einstellbar.

Die Spritze sitzt in ihren Aufnahmen mit einem gewissen Spiel. Außerdem sind die Schiebeeinrichtungen mit einem Spiel behaftet. Infolgedessen wird der Kolben nach dem Herausziehen bei konstanter Schrittweiteneinstellung in einem ersten Vorschiebeschritt um eine andere Distanz als in den nachfolgenden Schritten bewegt. Die beim Erstschritt ausgestoßene Flüssigkeitsmenge weicht also erheblich von dem Flüssigkeitsausstoß jedes nachfolgenden Schrittes ab. Für eine genaue Dosierung verwirft man deshalb in der Praxis das beim Erstschritt ausgestoßene Flüssigkeitsvolumen. Hierdurch geht Probenflüssigkeit verloren.

Die EP 0 679 439 B1 und US 5 591 408 beschreiben eine weiterentwickelte Repetierpipette, die zur Reduzierung des Verlustes an Probenflüssigkeit eine Konstantschritteinrichtung aufweist, die die Weite des ersten Abgabeschrittes auf einen konstanten Wert unabhängig von der Einstellung des Einstellelementes für die weiteren nachfolgenden Schritte festlegt.

Die DE 43 41 229 C2 und die US 5 620 660 schlagen ein besser für die Handbetätigung geeignetes Pipettensystem mit einer einfach axial in die Pipette einschiebbaren bzw. aus dieser entfernbaren Spritze vor. Dieses Pipettensystem hat eine einen Befestigungsabschnitt und einen Spritzenkolben aufweisende Spritze und eine Pipette, die in einem Pipettengehäuse eine Aufnahme für den Befestigungsabschnitt und einen Aufnahmekörper mit einer Kolbenaufnahme für den Spritzenkolben bzw. eine damit verbundene Kolbenstange aufweist. Ferner sind Befestigungseinrichtungen zum reversiblen bzw. lösbaren Fixieren von Befestigungsabschnitt und Spritzenkolben in den Aufnahmen und Kolbenstelleinrichtungen zum Verschieben des Aufnahmekörpers im Pipettengehäuse vorhanden. Der Befestigungsabschnitt und der Spritzenkolben sind durch Axialöffnungen ihrer Aufnahmen axial in ihre Befestigungspositionen schiebbar.

Die Befestigungseinrichtungen weisen radial zustellbare Greifeinrichtungen zum Fixieren des Befestigungsabschnitts und des Spritzenkolbens in den Befestigungspositionen auf. Die Greifeinrichtungen haben schwenkbar im Pipettengehäuse gelagerte Spritzengreifhebel und schwenkbar im Aufnahmekörper gelagerte Kolbengreifhebel. Die Spritzengreifhebel und die Kolbengreifhebel sind zweiarmig mit einem Greifarm und einem Betätigungsarm ausgeführt, wobei die Spritzengreifhebel an den Innenseiten ihrer Betätigungsarme Kontaktstellen aufweisen, die durch Betätigen ihrer Betätigungsarme außen gegen die Betätigungsarme der Kolbengreifhebel schwenkbar sind und die Kolbengreifhebel betätigen.

Hierdurch wird erreicht, dass die Spritze und die Pipette durch eine rein axiale Relativbewegung miteinander verbindbar und durch Betätigen der Betätigungsarme voneinander trennbar sind. Gemäß einer Ausgestaltung ist der Befestigungsabschnitt ein Spritzenflansch und gemäß einer weiteren Ausgestaltung weist der Spritzenkolben einen Kolbenbund zum Hintergreifen durch die Greifeinrichtungen auf.

Aus der DE 10 2005 023 203 und der US 2006263261 A1 ist eine Ausgestaltung der Pipette gemäß DE 43 41 229 C2 bekannt, bei der die Spritze durch Betätigen nur eines einzigen Auslösers von der Pipette lösbar ist.

Die kommerziellen Ausführungen Multipette^{®} (plus/stream/Xstream) dieses Dosiersystems der Firma Eppendorf AG umfassen Spritzen Combitips^{®} (plus), die einen Zylinder mit einem Kolbenlaufbereich an der Innenseite und einen Spritzenflansch am oberen Ende des Zylinders aufweisen. Die Spritzen werden mit verschiedenen Füllvolumina (0,1 ml, 0,2 ml, 0,5 ml, 1 ml, 2,5 ml, 5 ml und 10 ml) angeboten. Ferner umfasst das System Spritzen Combitips® (plus) mit Volumina von 25 ml und 50 ml. Diese Spritzen weisen einen großen Querschnitt auf, und sind über einen Adapter mit einer Repetierpipette verbindbar. Der Adapter ist an einem großen Durchmesser über eine Bajonettverbindung mit dem oberen Rand des Spritzenzylinders verbunden. Oben trägt er eine Hülse geringeren Durchmessers mit einem Flansch entsprechend dem Spritzenflansch der kleineren Spritzen. Über den Spritzenflansch wird der Adapter mit der Repetierpipette verbunden.

Das bekannte Dosiersystem ermöglicht genaue Dosierungen mit geringen Messabweichungen.

Davon ausgehend liegt der Erfindung die Aufgabe zugrunde, eine Spritze für den Gebrauch mit einer Dosiervorrichtung zur Verfügung zu stellen, die noch genauere Dosierungen ermöglicht.

Die Aufgabe wird durch eine Spritze mit den Merkmalen des Anspruches 1 gelöst.

Die erfindungsgemäße Spritze für den Gebrauch mit einer Dosiervorrichtung mit einer Aufnahme für einen Spritzenzylinder und einer axial verlagerbaren Kolbenaufnahme für einen Spritzenkolben hat
- einen Spritzenzylinder,
- der unten einen Boden mit einem Austritt,
- innen einen zylindrischen Kolbenlaufbereich und
- oben Mittel zum Halten in der Aufnahme hat,
- einen Spritzenkolben,
- der am Umfang eine umlaufende Kolbendichtung zum Abdichten am Kolbenlaufbereich und
- oben ein Kupplungsstück zum Einsetzen in die Kolbenaufnahme hat, und
- Anschlagmittel zwischen Spritzenzylinder und Spritzenkolben, die die Verlagerung des Spritzenkolbens zum Boden hin begrenzen, so dass der Spritzenkolben mit der Kolbendichtung nur bis auf einen bestimmten Abstand an den Boden annäherbar ist.

Der Erfindung liegt die überraschende Erkenntnis der Anmelderin zugrunde, dass bei der schrittweisen Abgabe von Probenflüssigkeit beim letzten Dosierschritt eine etwas größere Flüssigkeitsmenge abgegeben wird als bei den vorangehenden Abgabeschritten. Ferner haben umfangreiche Untersuchungen der Anmelderin zu dem überraschenden Ergebnis geführt, dass diese erhöhte Flüssigkeitsabgabe darauf beruht, dass sich beim letzen Dosierschritt der Boden des Spritzenzylinders um ein sehr geringes Maß nach innen in den Spritzenzylinder hinein verformt. Die Anmelderin hat herausgefunden, dass dies auf die radiale Aufweitung des Spritzenzylinders durch die an den Boden angenäherte Dichtlippe des Spritzenkolbens zurückzuführen ist. Infolgedessen kommt zur grundsätzlichen Verdrängung von Flüssigkeit durch die Verlagerung des Spritzenkolbens eine unbeabsichtigte zusätzliche Verdrängung von Flüssigkeit durch Verlagerung des Bodens hinzu. Die erfindungsgemäße Spritze überwindet diesen Effekt dadurch, dass sie Anschlagmittel aufweist, die die Verlagerung des Spritzenkolbens zum Boden hin so begrenzen, dass der Spritzenkolben mit der Kolbendichtung nur bis auf einen bestimmten Abstand an den Boden annäherbar ist. Dieser Abstand ist so gewählt, dass die Verformung des Bodens vermindert oder vermieden wird, die bei Annäherung der Kolbendichtung zu einem erhöhten Ausstoß von Flüssigkeit führt. Er beträgt vorzugsweise 1 mm und mehr. Hierdurch wird die Dosiergenauigkeit des letzten Abgabeschrittes verbessert und stimmt besser mit der Dosiergenauigkeit vorangehender Dosierschritte überein.

Gemäß einer Ausgestaltung weist der Spritzenkolben unterhalb der Kolbendichtung einen unteren Kolbenabschnitt auf, der in einen unteren Endbereich des Spritzenzylinders eingreift, wenn die Kolbendichtung bis auf den bestimmten Abstand an den Boden angenähert ist. Der untere Kolbenabschnitt füllt das Volumen im Spritzenzylinder unterhalb der Kolbendichtung weitgehend aus. Hierdurch werden Lufteinschlüsse zwischen Spritzenkolben und in die Spritze eingesogener Flüssigkeit weitgehend vermieden, die die Dosiergenauigkeit beeinträchtigen können. Bevorzugt ist der untere Kolbenabschnitt so dimensioniert, dass Lufteinschlüsse so wenig wie möglich auftreten, der Strömungswiderstand im entstehenden Spalt bei der Restmengenabgabe jedoch im akzeptablen Bereich bleibt, in dem die aufzuwendende Kraft bei Restmengenabgabe subjektiv nicht zu groß ist. Hierfür beträgt bevorzugt die Weite eines Spaltes zwischen dem unteren Kolbenabschnitt und dem unteren Endbereich des Spritzenzylinders wenige zehntel Millimeter.

Die Anschlagmittel zwischen Spritzenzylinder und Spritzenkolben können verschieden ausgeführt sein. Gemäß einer Ausgestaltung weisen die Anschlagmittel eine umlaufende Innenstufe des Spritzenzylinders auf, die in einem Abstand vom Boden angeordnet ist und die Verlagerung des Spritzenkolbens zum Boden hin begrenzt. Bevorzugt wird die Verlagerung des Spritzenkolbens dadurch begrenzt, dass sich die Dichtlippe an die Innenstufe des Spritzenzylinders anlegt. Bei Anlage des Spritzenkolbens an der Innenstufe befindet sich die Kolbendichtung in einem Abstand vom Boden, so dass die radiale Aufweitung des Spritzenzylinders im Bereich der Kolbendichtung keine oder eine gegenüber herkömmlichen Spritzen erheblich verminderte Verformung des Bodens nach innen zur Folge hat, die die Dosiergenauigkeit des letzten Dosierschrittes merklich beeinträchtigt.

Ein Spritzenzylinder mit einer Innenstufe weist unterhalb der Innenstufe einen unteren Endbereich mit einem vorzugsweise verringerten Innendurchmesser auf. Dies beruht auf der Herstellung des Spritzenzylinders durch Spritzgießen. Bevorzugt greift der Spritzenkolben in diesen unteren Endbereich mit einem unteren Kolbenabschnitt verringerten Durchmessers ein, wenn der Spritzenkolben so weit wie möglich in den Spritzenzylinder eingeschoben ist.

Gemäß einer Ausgestaltung weist der Spritzenzylinder zumindest angrenzend an den Boden außen axial über seinen Umfang erstreckte Rippen auf. Die axialen Rippen wirken einem radialen Aufweiten des Spritzenzylinders beim Einschieben des Spritzenkolbens und einem Hineinverformen des Bodens in den Spritzenzylinder entgegen. Hierdurch wird der Dosierfehler weiter verringert. Gemäß einer weiteren Ausgestaltung weist der Spritzenzylinder in dem unteren Endbereich mit verringertem Durchmesser außen axial erstreckte Rippen auf. Die axialen Rippen sind bevorzugt so bemessen, dass sie innerhalb der gedachten Verlängerung des Mantels des Spritzenzylinders im Kolbenlaufbereich liegen. Die Rippen stehen dann nicht über den Mantel des Spritzenzylinders im Kolbenlaufbereich nach außen vor. Gemäß einer weiteren Ausgestaltung sind die axialen Rippen gleichmäßig über den Umfang des Spritzenzylinders verteilt.

Ferner wird die Aufgabe durch eine Spritze mit den Merkmalen des Anspruches 7 gelöst.

Die erfindungsgemäße Spritze für den Gebrauch mit einer Dosiervorrichtung mit einer Aufnahme für einen Spritzenzylinder und einer axial verlagerbaren Kolbenaufnahme für einen Spritzenkolben hat
- einen Spritzenzylinder,
- der unten einen Boden mit einem Austritt,
- auf der Außenseite des Bodens radial erstreckte Rippen mit einer Höhe von zumindest 1 mm zumindest am äußeren Ende,
- innen einen zylindrischen Kolbenlaufbereich und
- oben Mittel zum Halten in der Aufnahme hat, und
- einen Spritzenkolben,
- der am Umfang eine umlaufende Kolbendichtung zum Abdichten am Kolbenlaufbereich und
- oben ein Kupplungsstück zum Einsetzen in die Kolbenaufnahme hat.

Bei der erfindungsgemäßen Spritze wird einer Verformung des Bodens in den Spritzenzylinder hinein beim letzten Dosierschritt dadurch entgegen gewirkt, dass der Boden durch radial erstreckte Rippen ausgesteift ist. Hierdurch wird eine Verdrängung von Flüssigkeit durch Verformung des Bodens zusätzlich zu einer Verdrängung von Flüssigkeit durch Verlagerung des Spritzenkolbens vermieden. Die Dosiergenauigkeit des letzten Dosierschrittes ist auch bei dieser Spritze verbessert.

Aus dem Markt bekannt sind zwar Spritzen ritips® professional z.B. 10 ml, die acht radial erstreckte Rippen auf dem Boden aufweisen. Diese radial erstreckten Rippen haben jedoch eine maximale Höhe von unter einem Millimeter am Austritt des Bodens und ihre Höhe nimmt radial nach außen auf den Wert Null am Außenumfang des Bodens ab. Diese Rippen sind offenbar aus ästhetischen Gründen angebracht. Aufgrund ihrer Bemessung und ihres Verlaufs sind die radial erstreckten Rippen ungeeignet, ein Hineinverformen des Bodens in den Spritzenzylinder beim letzten Abgabeschritt zu reduzieren.

Gemäß einer bevorzugten Ausgestaltung weist eine Spritze gemäß Anspruch 1 oder eines darauf bezogenen Unteranspruches die Merkmale einer Spritze von Anspruch 7 auf.

Gemäß einer weiteren Ausgestaltung weisen die Rippen zumindest am äußeren Ende eine Höhe von mindestens 3 mm, weiterhin bevorzugt von mindestens 5 mm auf. Bei dieser Ausgestaltung wird ein Hineinverformen des Bodens in den Spritzenzylinder beim letzten Dosierschritt weiter reduziert.

Die Erfindung bezieht Ausgestaltungen ein, bei denen die radialen Rippen eine konstante Höhe aufweisen oder die Höhe der radialen Rippen zum Austritt aus dem Boden hin zunimmt. Gemäß einer Ausgestaltung nimmt die Höhe der radialen Rippen zum Austritt hin ab. Hierdurch kann vermieden werden, dass die Rippen bei der Aufnahme und Abgabe von Probenflüssigkeit stören. Gemäß einer weiteren Ausgestaltung sind die radialen Rippen in Umfangsrichtung gleichmäßig über den Boden verteilt.

Gemäß einer weiteren Ausgestaltung weist der Spritzenzylinder eine den Boden seitlich übergreifende, zylindrische Verlängerung auf und erstrecken sich die radialen Rippen von der Innenseite dieser Verlängerung aus radial nach innen. Hierdurch wird der Boden weiter versteift.

Gemäß einer weiteren Ausgestaltung, die für sämtliche Erfindungsvarianten gilt, ist der Austritt in einer außen vom Boden vorstehenden Dosierspitze angeordnet. Bevorzugt laufen die radialen Rippen zur Dosierspitze hin flach aus.

Gemäß einer weiteren Ausgestaltung hat der Spritzenkolben unten eine Verdrängerspitze, die in die Dosierspitze eingreift, wenn der Spritzenkolben so weit wie möglich in den Spritzenzylinder eingeschoben ist. Bevorzugt ist dann ein geringer Spalt zwischen Verdrängerspitze und Dosierspitze vorhanden. Hierdurch wird einem Luftpolster zwischen Spritzenkolben und eingesogener Flüssigkeit entgegengewirkt.

Gemäß einer weiteren Ausgestaltung hat der Boden eine sich nach unten verjüngende, konische Form. Diese Form in Kombination mit radialen Rippen, deren Höhe zum Austritt hin abnimmt, vermeidet, dass die radialen Rippen beim Aufnehmen und Abgeben von Flüssigkeit stören.

Gemäß einer weiteren Ausgestaltung weist der Spritzenkolben einen Kolbenkopf auf, der über eine Stößelstange mit dem Kupplungsstück verbunden ist. Hierdurch wird eine materialsparende Ausführung des Spritzenkolbens erreicht. Hierzu weist gemäß einer weiteren Ausgestaltung die Stößelstange mehrere in Längsrichtung erstreckte Flügelstücke auf. Die Flügelstücke begünstigen eine stabile Stößelstange unter geringem Materialeinsatz.

Gemäß einer weiteren Ausgestaltung sind die Mittel zum Halten ein einteilig mit dem Spritzenzylinder verbundener Flansch und/oder ein mit dem Spritzenzylinder verbundener Adapter mit einem Flansch und/oder Mittel zum Verbinden des Spritzenzylinders mit einem Adapter. Der Adapter ist bevorzugt durch Bajonettverbindung oder Schraubverbindung mit dem Spritzenzylinder verbunden.

Gemäß einer weiteren Ausgestaltung ist die Kolbendichtung eine Dichtlippe am Umfang des Spritzenkolbens. Gemäß einer weiteren Ausgestaltung ist die Dichtlippe eine spitzwinkelig zum Umfang des Spritzenkolbens ausgerichtete Manschette. Gemäß einer weiteren Ausgestaltung ist die Dichtlippe zum Boden hin gerichtet.

Die erfindungsgemäße Spritze wird bevorzugt mit Füllvolumina von 10 ml, 12,5 ml, 25 ml und 50 ml hergestellt. Bei herkömmlichen Spritzen mit den genannten Füllvolumina kommt der Dosierfehler beim letzten Dosierschritt besonders zum Tragen.

Gemäß einer Ausgestaltung sind der Spritzenzylinder und/oder der Spritzenkolben jeweils einteilig aus Kunststoff hergestellt.

In dieser Anmeldung beziehen sich die Angaben "oben" und "unten" auf die bevorzugte Ausrichtung der Spritze beim Dosieren, bei der die Spritze senkrecht mit dem Austritt unten und dem Kupplungsstück oben gehalten wird.

Die Erfindung wird nachfolgend anhand der anliegenden Zeichnungen näher erläutert. In den Zeichnungen zeigen:

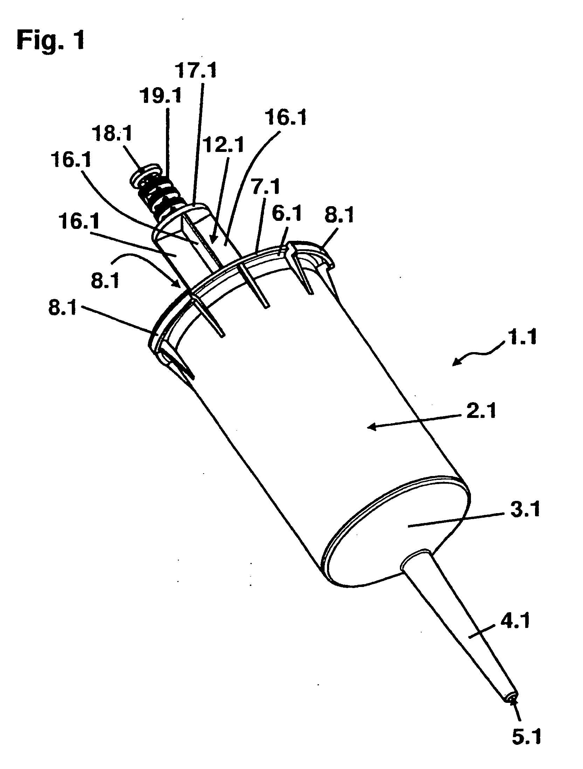

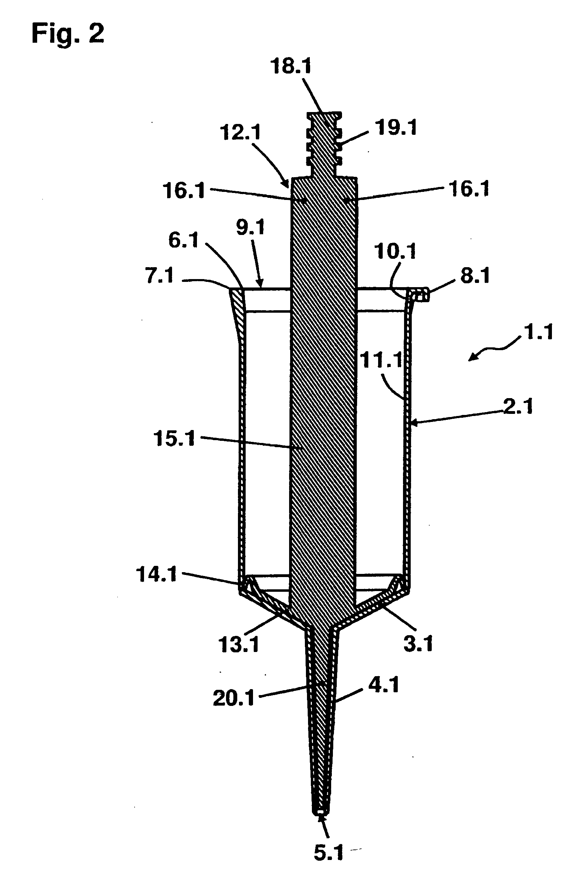

- Fig. 1 und 2: eine herkömmliche Spritze in einer Perspektivansicht schräg von unten und von der Seite (Fig. 1) und in einem Längsschnitt (Fig. 2);

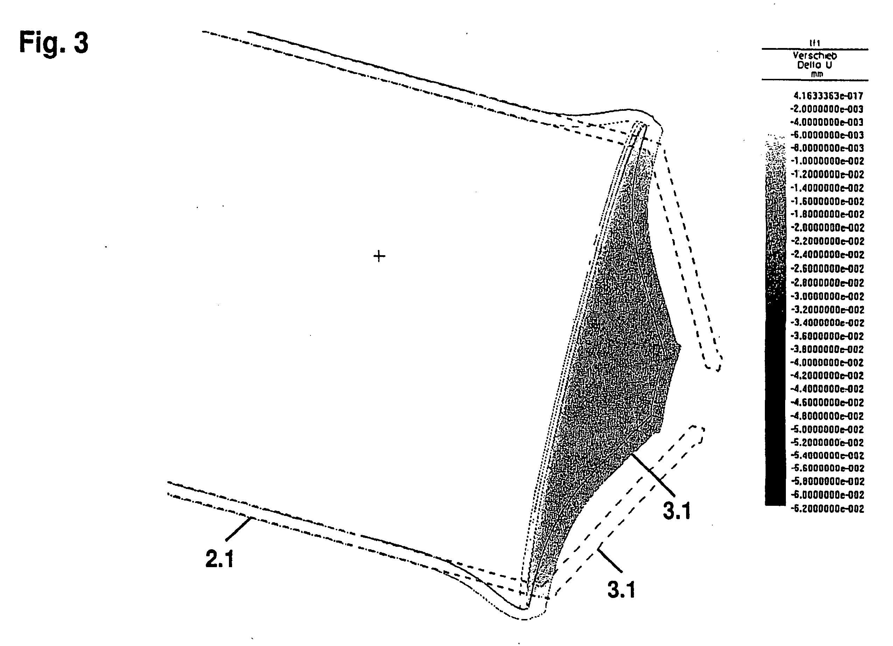

- Fig. 3: einen unteren Abschnitt des Spritzenzylinders von Fig. 1 bei nicht eingesetztem Spritzenkolben in gestrichelten Linien und bei vollständig eingeschobenem Spritzenkolben in ausgezogenen Linien in 200-facher Überzeichnung, wobei das Ausmaß der Verformung des Bodens durch Grautöne gemäß Legende angegeben ist;

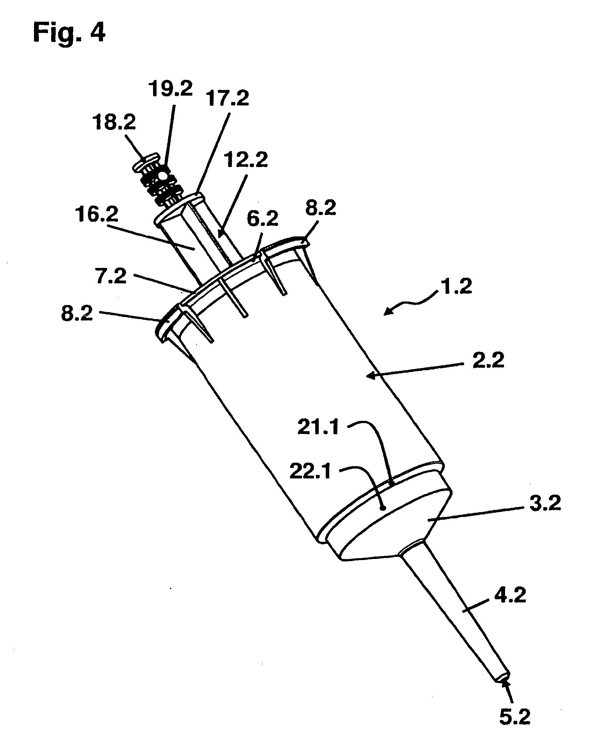

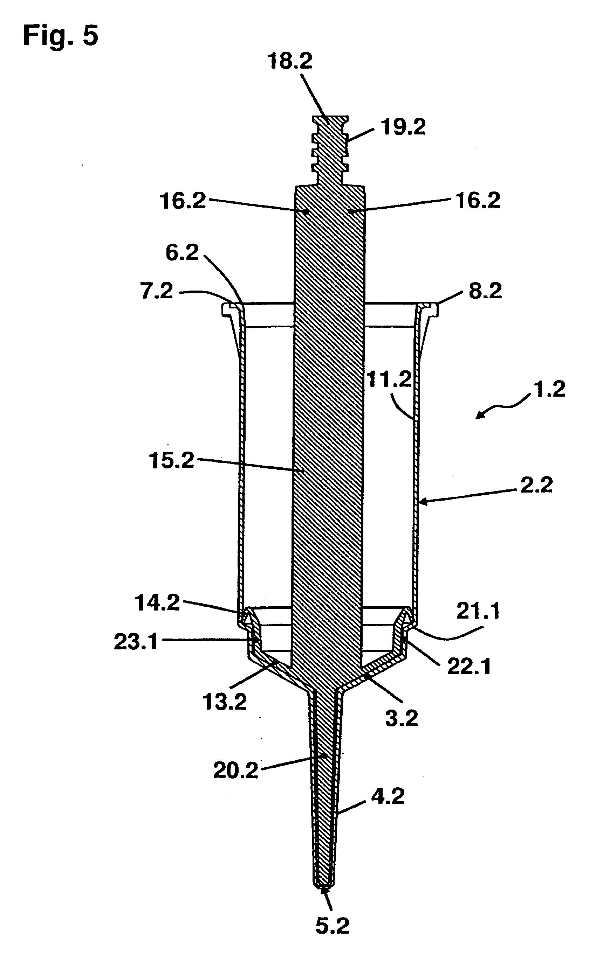

- Fig. 4 und 5: eine erfindungsgemäße Spritze mit einer Innenstufe des Spritzenzylinders als Anschlag für eine Dichtlippe des Kolbens in einer Perspektivansicht schräg von unten und von der Seite (Fig. 4) und in einem Längschnitt (Fig. 5);

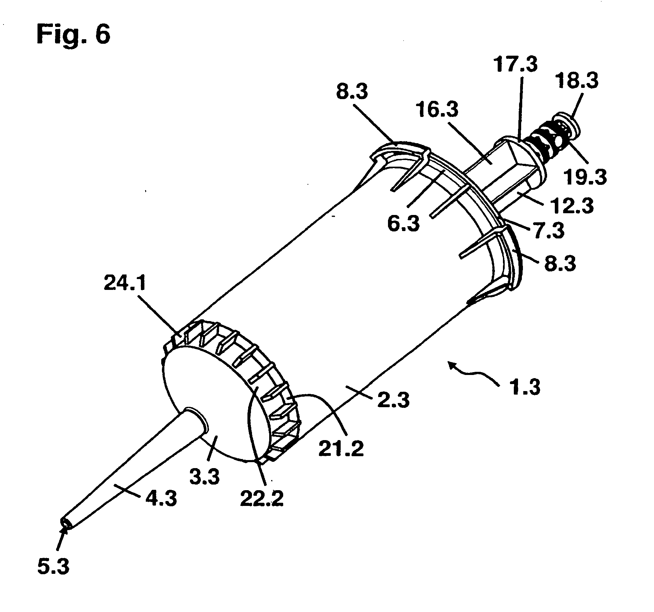

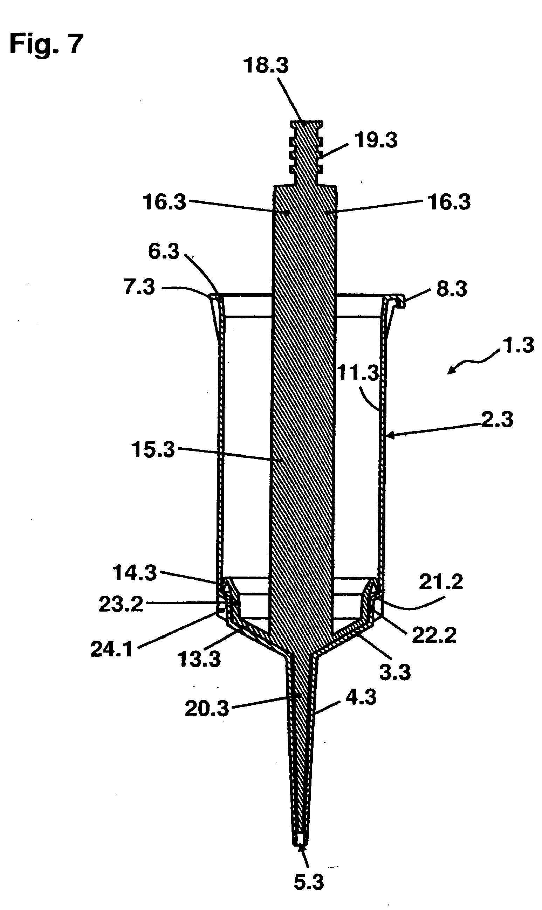

- Fig. 6 und 7: eine erfindungsgemäße Spritze mit einer Innenstufe des Spritzenzylinders als Anschlag für eine Dichtlippe des Kolbens mit axial erstreckten Rippen am Umfang des Spritzenzylinders in einer Perspektivansicht schräg von unten und von der Seite (Fig. 6) und in einem Längschnitt (Fig. 7);

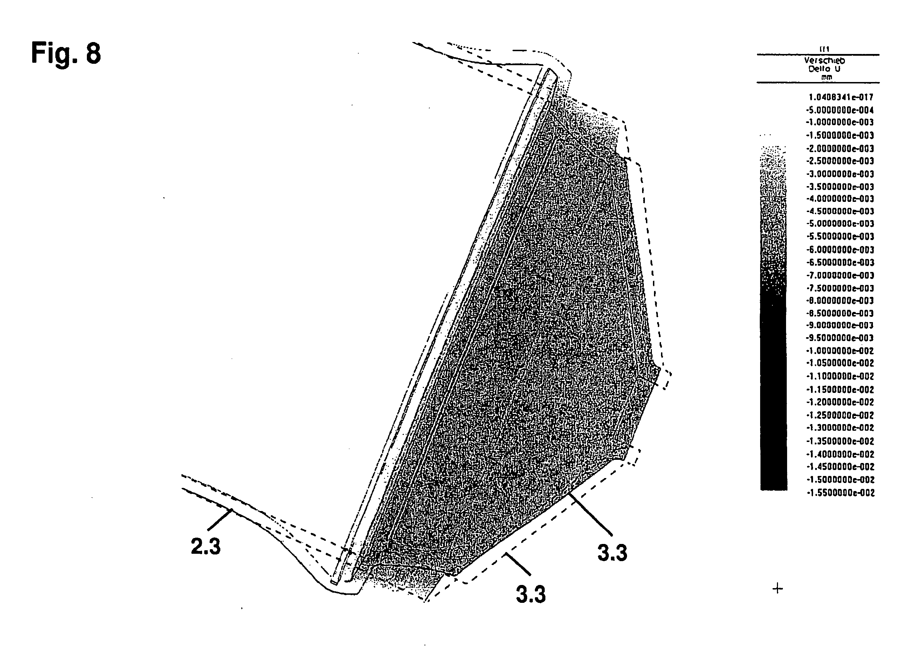

- Fig. 8: einen unteren Abschnitt des Spritzenzylinders der Spritze von Fig. 6 bei nicht eingesetztem Spritzenkolben in gestrichelten Linien und bei vollständig eingeschobenem Spritzenkolben in ausgezogenen Linien, wobei das Ausmaß der Verformung des Bodens durch Grautöne gemäß Legende angegeben ist;

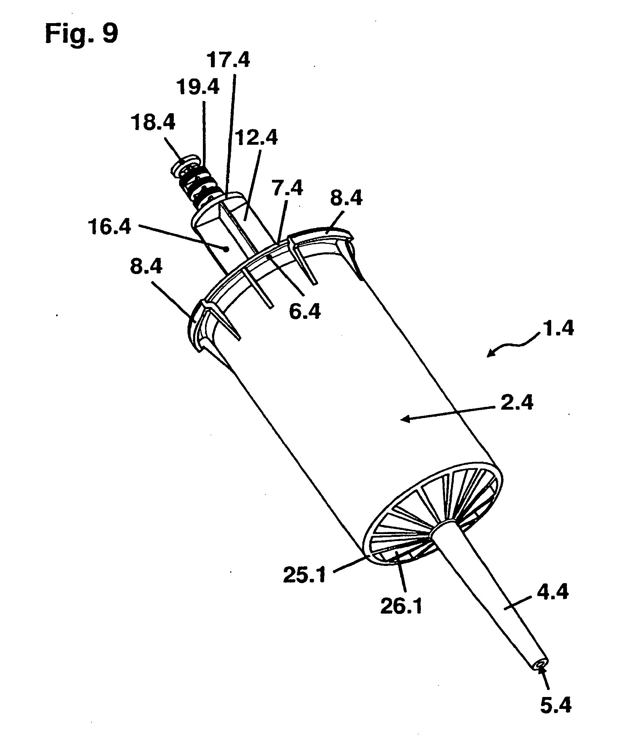

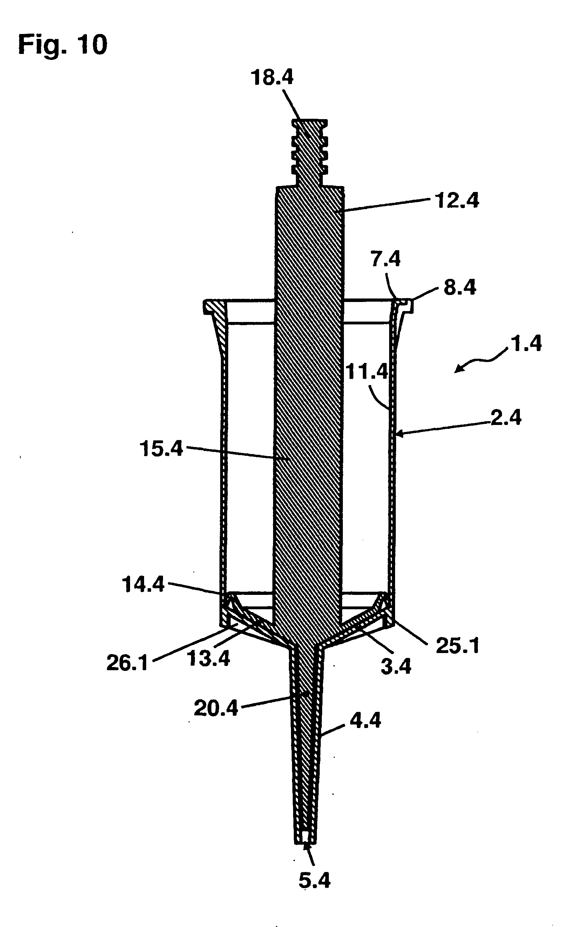

- Fig. 9 und 10: eine erfindungsgemäße Spritze mit radial erstreckten Rippen am Boden in einer Perspektivansicht schräg von unten und von der Seite (Fig. 9) und in einem Längsschnitt (Fig. 10);

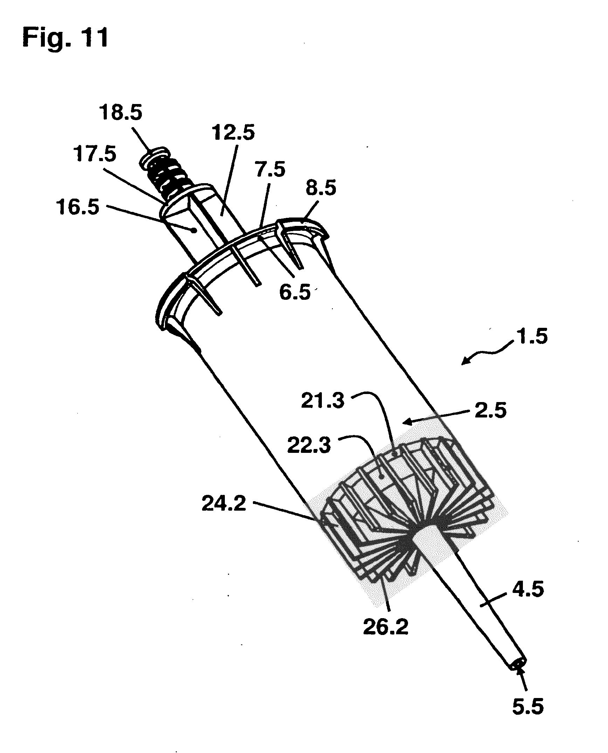

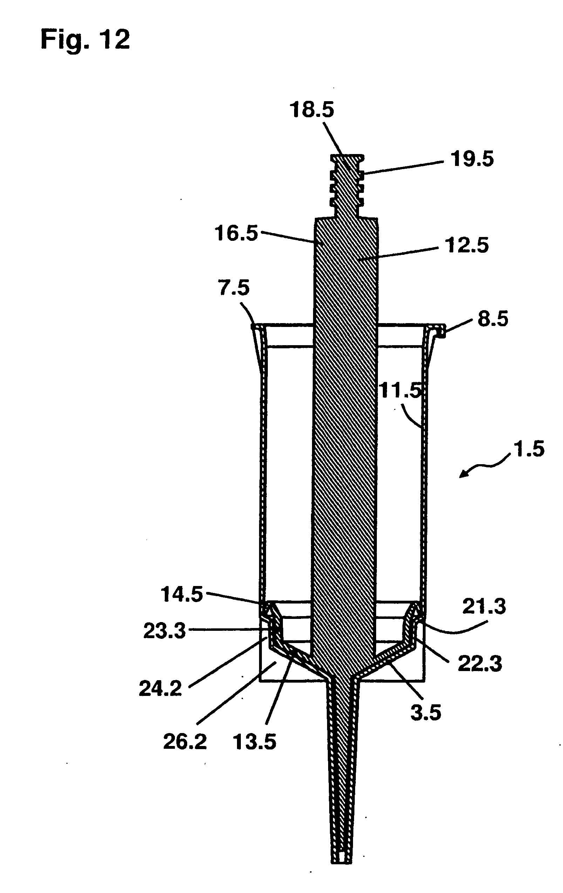

- Fig. 11 und 12: eine erfindungsgemäße Spritze mit Innenstufe des Spritzenzylinders als Anschlag für eine Dichtlippe des Spritzenkolbens, axial auf den Umfang des Spritzenzylinders erstreckten Rippen und in Fortsetzung der axial erstreckten Rippen radial auf dem Boden des Spritzenzylinders bis zum Austritt erstreckten Rippen in einer Perspektivansicht schräg von unten und von der Seite (Fig. 11) und in einem Längsschnitt (Fig. 12).

Bei der nachfolgenden Erläuterung sind einander entsprechende Teile verschiedener Ausführungsbeispiele mit mehreren Bezugsziffern versehen, die durch einen Punkt voneinander getrennt sind, wobei die vor dem Punkt stehenden Ziffern übereinstimmen und die hinter dem Punkt stehenden Ziffern das jeweilige Ausführungsbeispiel bezeichnen. Zusammenfassend sind einander entsprechende Teile verschiedener Ausführungsbeispiele auch allein mit den übereinstimmenden Ziffern bezeichnet.

Gemäß Fig. 1 und 2 hat eine herkömmliche Spritze 1.1 einen Spritzenzylinder 2.1, der unten einen konischen, sich nach unten verjüngenden Boden 3.1 aufweist. An der tiefsten Stelle des Bodens 3.1 befindet sich eine nach unten vorstehende, sich nach unten konisch verjüngende Dosierspitze 4.1. Die Dosierspitze 4.1 weist einen Austritt 5.1 für Flüssigkeit auf.

Der Spritzenzylinder 2.1 hat am oberen Rand 6.1 einen nach außen vorstehenden Flansch 7.1. Ferner stehen vom oberen Rand 6.1 drei Vorsprünge 8.1 noch weiter als der Flansch 7.1 nach außen vor. Die Vorsprünge 8.1 sind gleichmäßig über den Umfang des Spritzenzylinders verteilt. Die Vorsprünge 8.1 bilden eine Bajonettverbindung mit einem nicht gezeigten Adapter zum Halten des Spritzenzylinders 2.1 in einer nicht gezeigten Dosiervorrichtung.

Der obere Rand 6.1 umgibt eine Öffnung 9.1, die zum Einsetzen eines Spritzenkolbens dient. Angrenzend an die Öffnung 9.1 hat der Spritzenzylinder innen eine kurze Einführschräge 10.1. Daran schließt sich ein Laufbereich 11.1 für einen Spritzenkolben an, der sich bis zum Boden 3.1 erstreckt.

In den Spritzenzylinder 2.1 ist ein Spritzenkolben 12.1 eingesetzt. Der Spritzenkolben 12.1 hat unten einen im Wesentlichen konischen Kolbenkopf 13.1. Der Kolbenkopf 13.1 ist am äußeren Umfang mit einer im Querschnitt V-förmigen Dichtlippe 14.1 versehen. Die Dichtlippe 14.1 liegt mit ihrem äußeren Ende umfänglich innen an dem Kolbenlaufbereich 11.1 an. Die Dichtlippe 14.1 ist mit ihrem V-Querschnitt zum Boden 3.1 hin geöffnet.

Oben trägt der Kolbenkopf 13.1 eine Stößelstange 15.1, die über einen Großteil von vier streifenförmigen Flügelstücken 16.1 gebildet ist, die in einem horizontalen Querschnitt kreuzförmig zueinander ausgerichtet sind. Oben sitzt auf den Flügelstücken 16.1 eine Kreisscheibe 17.1. Darauf befindet sich ein Kupplungsstück 18.1, das eine Serie in Axialrichtung hintereinander angeordneter, kreisscheibenförmiger Vorsprünge 19.1 aufweist. Das Kupplungsstück 18.1 ist in eine Kolbenaufnahme einer nicht gezeigten Dosiervorrichtung einsetzbar und wird darin mittels geeigneter Haltemittel gehalten.

Ferner weist der Kolbenkopf 13.1 eine nach unten vorstehende, nach unten sich konisch verjüngende Verdrängerspitze 20.1 auf. Die Konizität des Kolbenkopfes 13.1 ist an die Konizität des Bodens 3.1 und die Konizität der Verdrängerspitze 20.1 an die Konizität der Dosierspitze 4.1 angepasst, ohne dass der Kolbenkopf auf dem Boden 3.1 und die Verdrängerspitze 20.1 auf der Dosierspitze 4.1 aufsitzt, wenn der Spritzenkolben 12.1 vollständig in den Spritzenzylinder 2.1 eingeschoben ist.

Der Spritzenzylinder 2.1 und der Spritzenkolben 12.1 werden jeweils einteilig aus einem Kunststoff spritzgegossen, wobei z. B. der Spritzenzylinder 2.1 aus Polypropylen und der Spritzenkolben 12.1 aus Polyethylen spritzgegossen ist.

Die Anmelderin hat herausgefunden, dass sich beim Einschieben des Spritzenkolbens 12.1 in den Spritzenzylinder 2.1 der Spritzenzylinder 2.1 über der Dichtlippe 14.1 radial aufweitet. Wenn der Spritzenkolben beim letzten Dosierschritt mit der Dichtlippe 14.1 auf den Boden 3.1 aufsitzt, wirkt sich dies dahingehend aus, dass sich der Spritzenboden 3.1 etwas nach innen in den Spritzenzylinder 2.1 hinein verformt.

Dies ist durch Fig. 3 veranschaulicht. Die in Fig. 3 gezeigte Verformung des Spritzenzylinders beruht auf FEM-Berechnungen (Berechnungen nach der Finite-Elemente-Methode). Bei diesen Berechnungen wurde lediglich die Verlagerung des Bodens bezüglich seiner Ausgangsposition in Axialrichtung betrachtet.

In Fig. 3 ist der Spritzenzylinder 2.1 in gestrichelten Linien im unverformten Zustand gezeigt, wenn der Spritzenkolben 12.1 nicht eingeschoben ist. Ferner ist der Spritzenzylinder 2.1 in ausgezogenen Linien in der Situation gezeigt, in der der Spritzenkolben 12.1 vollständig eingeschoben ist. Gut erkennbar ist, dass sich der Spritzenboden 3.1 in das Innere des Spritzenzylinders 2.1 hineinwölbt.

In Fig. 3 ist das Ausmaß der Verformung des Bodens 3.1 durch unterschiedliche Grautöne gekennzeichnet.
Die erfindungsgemäße Spritze 1.2 in Fig. 4 und 5 unterscheidet sich von der vorbeschriebenen dadurch, dass der Spritzenzylinder 2.2 in einem Abstand vom Boden 3.2 vom Innenumfang eine schmale Schulter 21.1 aufweist. Die Schulter 21.1 verjüngt sich konisch nach unten. Oberhalb der Schulter 21.1 befindet sich der Kolbenlaufbereich 11.2 der Spritze 1.2. Unterhalb der Schulter 21.1 hat der Spritzenzylinder 2.2 einen zylindrischen unteren Endbereich 22.1 verringerten Durchmessers.

Der Spritzenkolben 12.2 unterscheidet sich von dem zuvor beschriebenen 12.1 dadurch, dass der konische Kolbenkopf 13.2 einen geringeren Durchmesser mit einem vom oberen Rand vorstehenden zylindrischen Abschnitt 23.1 aufweist. Der obere Rand des zylindrischen Abschnittes 23.1 ist mit der V-förmigen Dichtlippe 14.2 verbunden, deren Form und Abmessungen wie bei der Dichtlippe 14.1 sind.

Gemäß der in Fig. 6 und 7 dargestellen Ausgestaltung hat die Spritze 1.3 die Besonderheit, dass der Spritzenzylinder 2.3 außen über dem unteren Endbereich 22.2 verringerten Durchmessers axial erstreckte Rippen 24.1 aufweist. Die axialen Rippen 24.1 erstrecken sich von der Außenseite der Innenstufe 21.2 bis beinahe zum Boden 3.3. Ihre Höhe ist so gewählt, dass sie nicht über den Mantel des Spritzenzylinders 2.3 hinaus stehen. Etwa 10 bis 30 axiale Rippen 24.1 sind gleichmäßig über den Umfang des Spritzenzylinders 2.3 verteilt.

Wenn bei den Spritzen 1.2 bzw. 1.3 der Spritzenkolben 12.2 bzw. 12.3 vollständig in den Spritzenzylinder 2.2 bzw. 2.3 eingeschoben ist, sitzt die Dichtlippe 14.2 bw. 14.3 auf der Innenstufe 21.1 bzw. 21.2 auf und verhindert ein weiteres Verlagern der Spritzenkolbens 12.2 bzw. 12.3 zum Boden 3.2 bzw. 3.3. Der Kolbenkopf 13.2 bzw. 13.3 ist durch einen kleinen Spalt vom Boden 3.2 bzw. 3.3 und die Verdrängerspitze 20.2 bzw. 20.3 ist durch einen kleinen Spalt von der Dosierspitze 4.2 bzw. 4.3 beabstandet. Die von der Dichtlippe 14.2 bzw. 14.3 bewirkte radiale Verformung wirkt sich somit nicht auf den Boden 3.2 bzw. 3.3 aus. Bei der Spritze 1.3 tragen hierzu auch die axialen Rippen 24.1 bei, die die Form des Spritzenzylinders 2.3 stabilisieren.

Fig. 8 zeigt die Ergebnisse einer weiteren FEM-Berechnung für die axiale Verformung des Spritzenzylinders 2.3 der Spritze 1.3 von Fig. 6 und 7. In gestrichelten Linien ist wiederum der unverformte Spritzenzylinder 2.3 gezeigt. In ausgezogenen Linien ist der Spritzenzylinder verformt durch den bis zum Anschlag vorgeschobenen Spritzenkolben 12.3 gezeigt. Der Maßstab ist derselbe wie bei Fig. 3. Die Verformungen des Spritzenbodens 3.3 sind äußerst gering. Die Grautöne zeigen, dass die äußeren Bereiche des Bodens 3.3 etwas stärker als die zentralen Bereiche verformt werden.

Die Fig. 9 und 10 zeigen eine weitere erfindungsgemäße Spritze 1.4. Diese unterscheidet sich von der Spritze 1.1 lediglich dadurch, dass der Mantel des Spritzenzylinders 2.4 einen Fortsatz 25.1 hat, der etwas über den Boden 3.4 hinaus steht. Im Bereich des Fortsatzes 25.1 ist die Wandstärke des Spritzenzylinders 2.4 größer als über dem Laufbereich 11.4.

Ferner weist der Spritzenzylinder 2.4 auf der Unterseite des Bodens eine Mehrzahl (z. B. 10 bis 30) radial erstreckter Rippen 26.1 auf. Die radialen Rippen 26.1 haben angrenzend an den Fortsatz 25.1 ihre größte Höhe. Diese beträgt mindestens 3 mm. Vom äußeren Umfang ausgehend nimmt die Höhe der Rippen 26.1 bis zur Dosierspitze 4.4 hin ab.

Die radialen Rippen 26.1 sind gleichmäßig über den Boden 3.4 verteilt.

Die radialen Rippen 26.1 bewirken eine Stabilisierung des Bodens 3.4, die ein Verformen des Bodens 3.4 stark reduziert, wenn die Dichtlippe 14.4 auf der Innenseite des Bodens 3.4. aufsetzt. Zur Reduzierung der Verformung des Bodens 3.4 trägt auch der Fortsatz 25.1 bei.

Fig. 11 und 12 zeigen eine weitere erfindungsgemäße Spritze 1.5, bei der der Spritzenzylinder 2.5 eine Innenstufe 21.3 aufweist und der Spritzenkolben 12.5 wie der Spritzenkolben 12.2 ausgebildet ist. Außerdem weist der Spritzenzylinder 2.5 außen (etwa 10 bis 30) axiale Rippen 24.2 und der Boden 3.5 außen radiale Rippen 26.2 auf. Die axialen Rippen 24.2 erstrecken sich bis zum Boden 3.5 und gehen dort in die radialen Rippen 26.2 über. Am äußeren Umfang des Bodens 3.5 sind die radialen Rippen deutlich höher als bei der Spritze 1.4.

Bei dieser Spritze 1.5 wird durch die Verlagerung der Kolbendichtung 14.5 vom Boden 3.5 weg und durch die Stabilisierung des Bodens 3.5 durch axiale Rippen 24.2 und radiale Rippen 26.2 die Verformung des Bodens 3.5 reduziert und einhergehende Dosierfehler vermieden.

## Patentansprüche

1. Spritze für den Gebrauch mit einer Dosiervorrichtung mit einer Aufnahme für einen Spritzenzylinder und einer axial verlagerbaren Kolbenaufnahme für einen Spritzenkolben mit
- einem Spritzenzylinder (2),
- der unten einen Boden (3) mit einem Austritt (5),
- innen einen zylindrischen Kolbenlaufbereich (11) und
- oben Mittel zum Halten (8) in der Aufnahme hat,
- einem Spritzenkolben (12),
- der am Umfang eine umlaufende Kolbendichtung (14) zum Abdichten am Kolbenlaufbereich (11) und
- oben ein Kupplungsstück (18) zum Einsetzen in die Kolbenaufnahme hat, und
- Anschlagmitteln (21) zwischen Spritzenzylinder (2) und Spritzenkolben (12), die die Verlagerung des Spritzenkolbens (12) zum Boden (3) hin begrenzen, so dass der Spritzenkolben (12) mit der Kolbendichtung (14) nur bis auf einen bestimmten Abstand an den Boden (3) annäherbar ist.

2. Spritze nach Anspruch 1, bei der der Kolben unterhalb der Kolbendichtung (14) einen unteren Kolbenabschnitt (23) aufweist, der in einen unteren Endbereich des Spritzenzylinders (2) eingreift, wenn die Kolbendichtung (14) bis auf einen bestimmten Abstand an den Boden (3) angenähert ist.

3. Spritze nach Anspruch 1 oder 2, bei der die Anschlagmittel (21) eine umlaufende Innenstufe (21) des Spritzenzylinders (2) aufweisen, die in einem Abstand vom Boden (3) angeordnet ist und die Verlagerung des Spritzenkolbens (12) zum Boden (3) hin begrenzt, und bei der der Spritzenzylinder (2) unterhalb der Innenstufe (21) einen unteren Endbereich (22) mit einem verringerten Innendurchmesser aufweist.

4. Spritze nach Anspruch 3, bei der der Spritzenkolben (12) unterhalb der Kolbendichtung (14) einen unteren Kolbenabschnitt (13, 23) verringerten Durchmessers aufweist, der in den unteren Endbereich (22) des Spritzenzylinders (2) eingreift, wenn die Kolbendichtung (14) bis auf den bestimmten Abstand an den Boden (3) angenähert ist.

5. Spritze nach einem der Ansprüche 1 bis 4, bei der der Spritzenzylinder (12) zumindest angrenzend an den Boden (3) außen axial auf seinem Umfang erstreckte Rippen (24) aufweist.

6. Spritze nach Anspruch 3 und 5, bei der der Spritzenzylinder (2) in dem unteren Endbereich (22) mit verringertem Innendurchmesser außen axial erstreckte Rippen (24) aufweist.

7. Spritze für den Gebrauch mit einer Dosiervorrichtung mit einer Aufnahme für einen Spritzenzylinder und einer axial verlagerbaren Kolbenaufnahme für einen Spritzenkolben mit
- einem Spritzenzylinder (2),
- der unten einen Boden (3) mit einem Austritt (5),
- auf der Außenseite des Bodens (3) radial erstreckte Rippen (26), mit einer Rippenhöhe von zumindest 1 mm zumindest am äußeren Ende,
- innen einen zylindrischen Kolbenlaufbereich (11) und
- oben Mittel zum Halten (8) in der Aufnahme hat, und
- einem Spritzenkolben (12),
- der am Umfang eine umlaufende Kolbendichtung (14) zum Abdichten am Kolbenlaufbereich (11) und
- oben ein Kupplungsstück (18) zum Einsetzen in die Kolbenaufnahme hat.

8. Spritze nach einem der Ansprüche 1 bis 6 mit den Merkmalen von Anspruch 7.

9. Spritze nach Anspruch 7 oder 8, bei der die radialen Rippen (26) zumindest am äußeren Ende eine Höhe von mindestens 3 Millimetern aufweisen.

10. Spritze nach einem der Ansprüche 7 bis 9, bei der die Höhe der radialen Rippen (26) zum Austritt (5) hin abnimmt.

11. Spritze nach einem der Ansprüche 1 bis 9, bei der der Boden (3) eine zylindrische oder sich nach unten verjüngende, konische Form hat.

12. Spritze nach einem der Ansprüche 1 bis 10, bei der der Austritt (5) in einer außen vom Boden (3) vorstehenden Dosierspitze (4) angeordnet ist.

13. Spritze nach Anspruch 11, bei der der Spritzenkolben (12) unten eine Verdrängerspitze (20) aufweist, die in die Dosierspitze (4) eingreift, wenn der Spritzenkolben (12) so weit wie möglich in den Spritzenzylinder (2) eingeschoben ist.

14. Spritze nach einem der Ansprüche 1 bis 13, bei der Spritzenkolben (12) einen Kolbenkopf (13) aufweist, der über eine Stößelstange (15) mit dem Kupplungsstück (18) verbunden ist.

15. Spritze nach Anspruch 14, bei der die Stößelstange (15) mehrere in Längsrichtung erstreckte Flügelstücke (16) umfasst.

16. Spritze nach einem der Ansprüche 1 bis 15, bei der die Mittel zum Halten (8) ein einteilig mit dem Spritzenzylinder (2) verbundener Flansch und/oder mit dem Spritzenzylinder (2) verbundener Adapter mit einem Flansch sind und/oder Mittel zum Verbinden des Spritzenzylinders (2) mit einem Adapter sind.

17. Spritze nach einem der Ansprüche 1 bis 16, bei der Spritzenzylinder (2) und/oder der Spritzenkolben (12) jeweils einteilig aus Kunststoff hergestellt sind.

## Claims

1. A syringe for use with a dosing device with a receiver for a syringe cylinder and an axially displaceable plunger receiver for a syringe plunger with
- a syringe cylinder (2),
- which has on the bottom a floor (3) with an outlet (5),
- a cylindrical plunger run area (11) on the inside and
- has on the top means for being held (8) in the receiver,
- a syringe plunger (12),
- which has on the perimeter a circumferential plunger seal (14) for sealing on the plunger run area (11) and
- a coupling piece (18) on the top for insertion into the plunger receiver and
- stop means (21) between the syringe cylinder (2) and the syringe plunger (12), which restrict the displacement of the syringe plunger (12) toward the floor (3) so that the syringe plunger (12) with the plunger seal (14) can only approach to a certain distance from the floor (3).

2. The syringe according to claim 1, in which the plunger below the plunger seal (14) has a lower plunger section (23), which engages into a bottom end area of the syringe cylinder (2) when the plunger seal (14) approaches to a certain distance from the floor (3).

3. The syringe according to claim 1 or 2, in which the stop means (21) has a circumferential inner step (21) of the syringe cylinder (2), which is arranged at a distance from the floor (3) and the displacement of the syringe plunger (12) towards the floor (3) is restricted, and in which the syringe cylinder (2) below the inner step (21) has a bottom end area (22) with a reduced inner diameter.

4. The syringe according to claim 3, in which the syringe plunger (12) below the plunger seal (14) has a bottom plunger section (13, 23) with a reduced diameter, which engages into a bottom end area (22) of the syringe cylinder (2) when the plunger seal (14) approaches to a certain distance from the floor (3).

5. The syringe according to one of claims 1 through 4, in which the syringe cylinder (12) has at least adjacent to the floor (3) ribs (24) extending axially to its perimeter on the outside.

6. The syringe according to claim 3 and 5, in which the syringe cylinder (2) in the bottom end area (22) with reduced inner diameter has axially extending ribs (24) on the outside.

7. A syringe for use with a dosing device with a receiver for a syringe cylinder and an axially displaceable plunger receiver for a syringe plunger with
- a syringe cylinder (2),
- which has on the bottom a floor (3) with an outlet (5),
- on the outside of the floor (3) radially extending ribs (26) with a rib height of at least 1 mm at least on the outer end,
- a cylindrical plunger run area (11) on the inside and
- has on the top means for being held (8) in the receiver and
- a syringe plunger (12),
- which has on the perimeter a circumferential plunger seal (14) for sealing on the plunger run area (11) and
- a coupling piece (18) on the top for insertion into the plunger receiver.

8. The syringe according to one of claims 1 through 6 with the characteristics of claim 7.

9. The syringe according to claim 7 or 8, in which the radial ribs (26) have at least on the outer end a height of at least 3 millimeters.

10. The syringe according to one of claims 7 through 9, in which the height of the radial ribs (26) decreases towards the outlet (5).

11. The syringe according to one of claims 1 through 9, in which the floor (3) has a cylindrical or downward tapering, conical shape.

12. The syringe according to one of claims 1 through 10, in which the outlet (5) is arranged in a dosing tip (4) protruding from the floor (3).

13. The syringe according to claim 11, in which the syringe plunger (12) has a displacement tip (20), which engages into the dosing tip (4) when the syringe plunger (12) is pushed as far as possible into the syringe cylinder (2).

14. The syringe according to one of claims 1 through 13, in which the syringe plunger (12) has a plunger head (13), which is connected with the coupling piece (18) via a push rod (15).

15. The syringe according to claim 14, in which the push rod (15) comprises several wing pieces (16) extending in the longitudinal direction.

16. The syringe according to one of claims 1 through 15, in which the means for holding (8) are a flange connected as one piece with the syringe cylinder (2) and/or an adapter connected with the syringe cylinder (2) with a flange and/or means for connecting the syringe cylinder (2) with an adapter.

17. The syringe according to one of claims 1 through 16, in which the syringe cylinder (2) and/or the syringe plunger (12) are each produced as a single piece made of plastic.

## Revendications

1. Seringue pour l'utilisation à l'aide d'un dispositif de dosage avec un logement pour un cylindre de seringue et un logement de piston déplaçable axialement pour un piston de seringue, avec
- un cylindre de seringue (2),
- qui a en bas un fond (3) avec une sortie (5),
- intérieurement une zone de mouvement de piston (11) cylindrique, et
- en haut des moyens pour la retenue (8) dans le logement,
- avec un piston de seringue, (12)
- qui a à la périphérie un joint d'étanchéité de piston (14) périphérique pour réaliser une étanchéité sur la zone de mouvement de piston (11) et
- en haut une pièce d'accouplement (18) pour l'introduction dans le logement de piston, et
- avec des moyens de butée (21) entre le cylindre de seringue (2) et le piston de seringue (12), qui limitent le déplacement du piston de seringue (12) vers le fond (3) de telle sorte que le piston de seringue, (12) avec le joint d'étanchéité de piston (14) ne peut être rapproché du fond (3) que jusqu'à une distance définie.

2. Seringue selon la revendication 1, dans laquelle le piston présente, au-dessous du joint d'étanchéité de piston (14), un tronçon de piston inférieur (23) qui engrène dans une zone d'extrémité inférieure du cylindre de seringue, (2) quand le joint d'étanchéité de piston (14) est rapproché du fond (3) jusqu'à une distance définie.

3. Seringue selon la revendication 1 ou 2, dans laquelle les moyens de butée (21) présentent un gradin intérieur (21) périphérique du cylindre de seringue (2) qui est disposé à une distance du fond (3) et qui limite le déplacement du piston de seringue, (12) vers le fond (3), et dans laquelle le cylindre de seringue (2) présente, au-dessous du gradin intérieur (21), une zone d'extrémité inférieure (22) avec un diamètre intérieur réduit.

4. Seringue selon la revendication 3, dans laquelle le piston de seringue (12) présente, au-dessous du joint d'étanchéité de piston (14), un tronçon de piston inférieur (13, 23) d'un diamètre réduit qui engrène dans la zone d'extrémité inférieure (22) du cylindre de seringue (2) quand le joint d'étanchéité de piston (14) est rapproché du fond (3) jusqu'à une distance définie.

5. Seringue selon une des revendications 1 à 4, dans laquelle le cylindre de seringue, (12) présente, au moins de façon adjacente au fond (3), des nervures (24) s'étendant à l'extérieur axialement sur sa périphérie.

6. Seringue selon les revendications 3 et 5, dans laquelle le cylindre de seringue, (2) présente, dans la zone d'extrémité inférieure (22) de diamètre réduit, des nervures (24) s'étendant à l'extérieur axialement.

7. Seringue pour l'utilisation à l'aide d'un dispositif de dosage avec un logement pour un cylindre de seringue et un logement de piston déplaçable axialement pour un piston de seringue, avec
- un cylindre de seringue (2),
- qui a en bas un fond (3) avec une sortie (5),
- des nervures (26) s'étendant radialement sur le côté extérieur du fond (3), avec une hauteur de nervure d'au moins 1 mm au moins à l'extrémité extérieure,
- à l'intérieur une zone de mouvement de piston (11) cylindrique, et
- en haut des moyens pour la retenue (8) dans le logement, et
- avec un piston de seringue (12),
- qui a à la périphérie un joint d'étanchéité de piston (14) périphérique pour réaliser l'étanchéité sur la zone de mouvement de piston (11) et
- en haut une pièce d'accouplement (18) pour l'introduction dans le logement de piston.

8. Seringue selon une des revendications 1 à 6, avec les caractéristiques de la revendication 7.

9. Seringue selon la revendication 7 ou 8, dans laquelle les nervures (26) radiales présentent, au moins à l'extrémité extérieure, une hauteur d'au moins 3 millimètres.

10. Seringue selon une des revendications 7 à 9, dans laquelle la hauteur des nervures (26) radiales diminue vers la sortie (5).

11. Seringue selon une des revendications 1 à 9, dans laquelle le fond (3) a une forme cylindrique ou une forme conique se rétrécissant vers le bas.

12. Seringue selon une des revendications 1 à 10, dans laquelle la sortie (5) est disposée dans une pointe de dosage (4) dépassant extérieurement du fond (3).

13. Seringue selon la revendication 11, dans laquelle le piston de seringue (12) présente en bas une pointe de déplacement (20) qui engrène dans la pointe de dosage (4) quand le piston de seringue (12) est poussé le plus loin possible dans le cylindre de seringue (2).

14. Seringue selon une des revendications 1 à 13, dans laquelle le piston de seringue, (12) présente une tête de piston (13) qui est raccordée à la pièce d'accouplement (18) par le biais d'une tige de poussoir (15).

15. Seringue selon la revendication 14, dans laquelle la tige de poussoir (15) comprend plusieurs pièces d'ailette (16) s'étendant dans la direction longitudinale.

16. Seringue selon une des revendications 1 à 15, dans laquelle les moyens pour la retenue (8) sont une bride raccordée d'une seule pièce avec le cylindre de seringue, (2) et/ou un adaptateur raccordé au cylindre de seringue, (2) avec une bride et/ou des moyens pour le raccordement du cylindre de seringue (2) à un adaptateur.

17. Seringue selon une des revendications 1 à 16, dans laquelle le cylindre de seringue (2) et/ou le piston de seringue (12) sont respectivement fabriqués d'une pièce en matière plastique.
